# FASCICULE DE BREVET EUROPEEN

(11) **EP 1 142 609 B1**
(45) Date de publication et mention de la délivrance du brevet: **24.01.2007**
(21) Numéro de dépôt: 01400890.8
(22) Date de dépôt: 06.04.2001
(51) Int. Cl.: A61N 1/37, A61B 5/04

(54) **Procédé d'échantillonnage d'un paramètre cardiaque dans un dispositif médical implantable actif**
Verfahren zur Abstatung von einem Herzparameter in einer implantierbaren aktiven medizinischen Vorrichtung
Process for sampling a cardiac parameter in an implantable active medical device

(30) Priorité: 07.04.2000 FR 0004510
(43) Date de publication de la demande: 10.10.2001
(73) Titulaire: ELA MEDICAL, 92541 Montrouge (FR)
(72) Inventeur: Dal Molin, Renzo, 92320 Chatillon (FR)
(74) Mandataire: Dupuis-Latour, Dominique

(56) Documents cités:
- EP-A- 0 426 567
- US-A- 5 847 569

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

L'invention s'applique tout particulièrement à la mesure de l'impédance intracardiaque, par exemple l'impédance interventriculaire (entre ventricule droit et ventricule gauche) dans un dispositif de type multisite, qui est un paramètre utile pour piloter notamment la resynchronisation des ventricules ou bien l'impédance transvalvulaire (entre oreillette et ventricule d'un même côté du coeur) ou encore l'impédance transseptale oblique (entre oreillette droite et ventricule gauche). Ces données sont corrélées au débit cardiaque et permettent d'obtenir notamment une indication de la fraction d'éjection, qui est le paramètre hémodynamique de référence pour l'optimisation de la stimulation sur les différents sites d'un dispositif multisite.

Cette application n'est cependant pas limitative, et l'invention s'applique à la mesure d'autres grandeurs intracardiaques, par exemple à l'analyse d'un électrocardiogramme intracardiaque, ou applications analogues.

Jusqu'à présent, le paramètre à déterminer est échantillonné à partir d'un signal recueilli par l'implant, typiquement avec un pas d'échantillonnage de 16 ms, cet échantillonnage étant répété à chaque cycle.

L'un des buts de l'invention est de proposer un procédé d'échantillonnage perfectionné permettant de déterminer le paramètre recherché en diminuant le nombre d'échantillons recueillis, et par voie de conséquence la consommation de l'implant, sans pour autant altérer la qualité du résultat obtenu (lui-même lié à la fréquence d'échantillonnage, qui doit être suffisamment élevée).

L'invention propose à cet effet de déterminer le paramètre cardiaque considéré en exécutant de manière répétée au cours d'une pluralité de cycles cardiaques successifs les étapes consistant à détecter l'instant de survenue d'un événement cardiaque, notamment un événement ventriculaire, et opérer ensuite un échantillonnage dudit signal au cours du cycle cardiaque avec un pas d'échantillonnage constant et un décalage temporel prédéterminé entre l'événement cardiaque détecté et le premier échantillon, ce décalage temporel étant un décalage progressif variable d'un cycle au suivant.

De préférence, la durée maximale du décalage temporel variable est toujours inférieure à la durée d'un cycle courant, et croît d'un incrément constant d'un cycle au suivant.

Une fois opérés les échantillonnages sur un nombre donné de cycles successifs, les échantillons recueillis peuvent être lissés pour donner une grandeur représentative dudit paramètre cardiaque.

On va maintenant décrire un exemple de mise en oeuvre de l'invention, en référence à la figure unique annexée qui est un schéma montrant la distribution relative des instants d'échantillonnage au cours de cycles cardiaques successifs.

L'une des considérations à l'origine de l'invention est le fait que les paramètres que l'on souhaite mesurer, comme l'impédance intracardiaque, évoluent comme le débit sanguin, donc de façon relativement lente à l'échelle de la durée d'un cycle cardiaque si l'état du patient est relativement constant et en l'absence de troubles du rythme fréquents. Il est ainsi possible d'étaler la mesure sur plusieurs cycles cardiaques successifs sans pour autant altérer notablement la qualité de restitution des variations de cette grandeur.

En réduisant le nombre d'échantillons pris sur chaque cycle, on réduira dans les mêmes proportions la consommation des circuits de l'implant opérant le recueil et le traitement (conversion analogique/numérique, mémorisation, lissage, etc.) du paramètre considéré.

Ainsi, au lieu d'effectuer une mesure sur un cycle cardiaque à chaque cycle, il est par exemple possible d'étaler la mesure sur huit cycles en prenant huit fois moins d'échantillons à chaque cycle, et diviser ainsi la consommation également par huit.

Mais pour pouvoir combiner entre elles les mesures recueillies au cours de cycles différents, il est nécessaire de disposer d'une référence temporelle précise, sur laquelle seront synchronisées les prises d'échantillons.

On choisit à cet effet comme référence temporelle l'instant de survenue d'un événement cardiaque, c'est-à-dire que l'on prend comme origine des temps l'instant de détection d'une dépolarisation, ou l'instant de stimulation, d'une cavité cardiaque, par exemple d'un ventricule, généralement le ventricule droit.

Au cours du premier cycle, un échantillonnage est opéré avec un pas d'échantillonnage constant, par exemple 64 ms, et le premier échantillon est pris avec un décalage temporel Δt₁ donné par rapport à l'instant de survenue de l'événement, par exemple un décalage de 8 ms. L'échantillonnage est poursuivi pendant une durée donnée, par exemple pendant une durée de 320 ms suivant l'événement ventriculaire.

En d'autres termes, pour le premier cycle, l'échantillonnage est opéré à 8, 72, 136, 200 et 264 ms après l'événement ventriculaire.

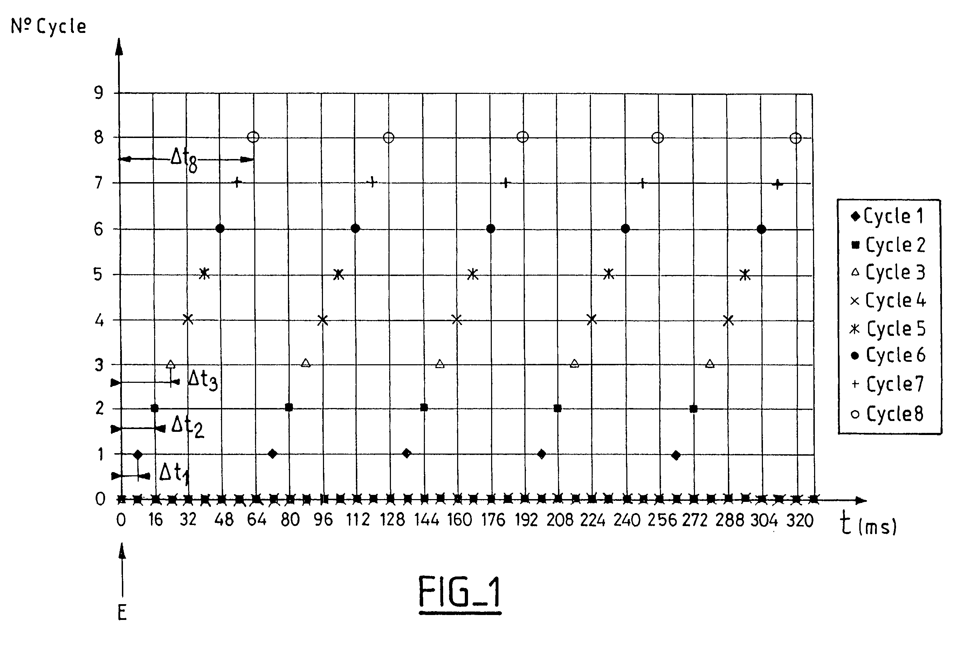

Ces cinq points d'échantillonnage sont illustrés sur la figure 1 par des losanges, l'origine des temps étant l'événement ventriculaire E.

On notera que le pas d'échantillonnage choisi, ici de 64 ms, est très supérieur au pas d'échantillonnage des dispositifs de l'art antérieur, typiquement de 8 ou 16 ms, entraînant de ce fait une réduction corrélative de la consommation de l'implant.

Au deuxième cycle, on effectue un échantillonnage avec le même pas de 64 ms que précédemment, mais avec un décalage temporel Δt₂ = 16 ms par rapport à l'événement ventriculaire E, au lieu de Δt₁ = 8 ms. Les échantillons sont donc recueillis à 16, 80, 144, 208 et 272 ms après l'événement E, comme illustré par les carrés sur la figure 1.

On procède de la même façon aux cycles suivants, en gardant le même pas d'échantillonnage mais en augmentant progressivement le décalage temporel Δt₃, Δt₄ ... Δt₈, avec un incrément constant de 8 ms (c'est-à-dire que Δt₁ = 8 ms, Δt₂ = 16 ms ... Δt₈ = 64 ms).

Au bout du huitième cycle cardiaque, si l'on a choisi de manière appropriée l'incrément Δtᵢ-Δtᵢ₋₁ du décalage temporel Δt_{i,} on aura balayé la majeure partie de la zone du cycle cardiaque à étudier avec un "effet stroboscopique", en ne prélevant qu'un nombre réduit d'échantillons à chaque cycle.

Au bout de huit cycles on dispose ainsi de quarante échantillons, qui permettront de déterminer la valeur et l'évolution du paramètre considéré.

Le paramètre pourra ainsi être évalué au bout de huit cycles, puis au bout de seize cycles, etc. ; il pourra être également réévalué plus fréquemment, par exemple à chaque cycle avec lissage sur les huit derniers cycles, ou tout autre technique analogue.

L'incrément Δtᵢ-Δtᵢ₋₁ entre deux décalages temporels successifs (qui est dans l'exemple décrit de 8 ms) est choisi suffisamment faible pour permettre de bien voir les détails de la variation du signal à l'intérieur du cycle cardiaque (théorie de Shannon appliquée à l'échantillonnage d'un signal), et pour éviter un chevauchement des mesures effectuées au cours des cycles successifs, c'est-à-dire par exemple éviter que le premier échantillon recueilli au huitième cycle (à E+64 ms) ne soit postérieur au deuxième échantillon recueilli au cours du premier cycle (à E+72 ms).

L'incrément Δtᵢ-Δtᵢ₋₁ sera cependant choisi suffisamment grand pour couvrir toute la zone à échantillonner : dans l'exemple illustré, il n'existe ainsi qu'un faible intervalle entre le premier échantillon du huitième cycle (à E+64 ms) et le deuxième échantillon du premier cycle (à E+72 ms). Ceci permet de couvrir de façon complète et homogène la zone [E ; E+320 ms], c'est-à-dire les 320 premières millisecondes d'un cycle cardiaque (la durée totale d'un cycle cardiaque étant de l'ordre de 860 ms à 70 cpm).

Cet incrément Δtᵢ-Δtᵢ₋₁ n'est pas nécessairement fixe ; il peut être adapté dynamiquement en fonction des besoins ou de certaines conditions, par exemple diminué en cas d'augmentation de la fréquence cardiaque, et inversement.

L'incrément Δtᵢ-Δtᵢ₋₁ ainsi que le décalage initial Δt₁ peuvent également être choisis de manière à concentrer l'analyse du paramètre considéré sur une "fenêtre" de mesure particulière à l'intérieur du cycle cardiaque, ce qui ne pose pas de difficulté particulière puisqu'au cours des cycles successifs l'échantillonnage reste toujours synchronisé sur l'événement ventriculaire E.

## Revendications

1. Un procédé de détermination d'un paramètre cardiaque, notamment d'une impédance intracardiaque ou d'un électrocardiogramme intracardiaque, à partir d'un signal recueilli par un dispositif médical implantable actif tel qu'un stimulateur cardiaque, défibrillateur, cardioverteur et/ou dispositif multisite,
**caractérisé en ce qu'**il consiste à exécuter de manière répétée au cours d'une pluralité de cycles cardiaques successifs (CYCLE 1 ... CYCLE 8) les étapes consistant à :
- détecter l'instant (E) de survenue d'un événement cardiaque, et
- opérer ensuite un échantillonnage dudit signal au cours du cycle cardiaque avec un pas d'échantillonnage constant et un décalage temporel prédéterminé (Δt₁ ... Δt₈) entre l'événement cardiaque détecté et le premier échantillon, ce décalage temporel étant un décalage progressif variable d'un cycle au suivant.

2. Le procédé de la revendication 1, dans lequel la durée maximale du décalage temporel variable (Δtᵢ) est toujours inférieure à la durée d'un cycle courant.

3. Le procédé de la revendication 1, dans lequel la durée du décalage temporel variable (Δtᵢ) croît d'un incrément constant (Δtᵢ-Δtᵢ₋₁) d'un cycle au suivant.

4. Le procédé de la revendication 1, dans lequel, une fois opérés les échantillonnages sur un nombre donné de cycles successifs, les échantillons recueillis sont lissés pour donner une grandeur représentative dudit paramètre cardiaque.

5. Le procédé de la revendication 1, dans lequel ledit événement cardiaque est un événement ventriculaire.

## Claims

1. A method for determining a heart parameter, in particular an intracardiac impedance or an intracardiac electrocardiogram, on the basis of a signal recorded by an active implantable medical device such as a pacemaker, defibrillator, cardioverter and/or multisite de vice,
**characterised in that** it consists in repeatedly executing during a plurality of successive cardiac cycles (CYCLE 1 CYCLE 8) the steps consisting in:
- detecting the moment (E) of occurrence of a cardiac event, and,
- subsequently, performing a sampling of said signal during the cardiac cycle with a constant sampling step and a predetermined temporal offset (Δt₁ ... Δt₈) between the detected cardiac event and the first sample, this temporal offset being a progressive offset varying from one cycle to the next.

2. The method of claim 1, wherein the maximum duration of the variable temporal offset (Δtᵢ) is always lower than the duration of the current cycle.

3. The method of claim 1, wherein the duration of the variable temporal offset (Δtᵢ) increases by a constant increment (Δtᵢ - Δtᵢ₋₁) from one cycle to the next.

4. The method of claim 1, wherein, once the sampling over a given number of successive cycles has been performed, the recorded samples are smoothened to yield a quantity representative for said cardiac parameter.

5. The method of claim 1, wherein said cardiac event is a ventricular event.

## Patentansprüche

1. Verfahren zur Bestimmung eines Herzparameters, insbesondere einer intrakardialen Impedanz oder eines intrakardialen Elektrokardiogramms, anhand eines Signals, das durch eine aktive implantierbare medizinische Vorrichtung wie bspw. einen Herzschrittmacher, Defibrillator, Kardioverter und/oder eine Multisite-Vorrichtung aufgenommen wird,
**dadurch gekennzeichnet, dass** es darin besteht, wiederholt im Verlauf einer Mehrzahl von aufeinander folgenden Herzzyklen (CYCLE 1 ... CYCLE 8) die Schritte auszuführen, die darin bestehen:
- den Zeitpunkt (E) des Auftretens eines Herzereignisses zu erfassen, und
- anschließend eine Abtastung des Signals während des Herzzyklus mit einem konstanten Abtastschritt und einer vorbestimmten zeitlichen Verschiebung (Δt₁ ... Δt₈) zwischen dem erfassten Herzereignis und dem ersten Abtastwert durchzuführen, wobei diese zeitliche Verschiebung eine progressive, von einem Zyklus zum nächsten variable Verschiebung ist.

2. Verfahren nach Anspruch 1, bei welchem die maximale Dauer der variablen zeitlichen Verschiebung (Δtᵢ) stets geringer ist als die Dauer eines laufenden Zyklus.

3. Verfahren nach Anspruch 1, bei welchem die Dauer der variablen zeitlichen Verschiebung (Δtᵢ) von einem Zyklus zum nächsten um ein konstantes Inkrement (Δtᵢ - Δtᵢ₋₁) steigt.

4. Verfahren nach Anspruch 1, bei welchem die aufgenommenen Abtastwerte, nachdem die Abtastungen über eine gegebene Anzahl von aufeinander folgenden Zyklen durchgeführt wurden, geglättet werden, um einen repräsentativen Wert des Herzparameters zu ergeben.

5. Verfahren nach Anspruch 1, bei welchem das Herzereignis ein Herzkammerereignis ist.
